# EUROPÄISCHE PATENTSCHRIFT

(11) **EP 0 496 060 B1**
(45) Veröffentlichungstag und Bekanntmachung des Hinweises auf die Patenterteilung: **03.01.1996**
(21) Anmeldenummer: 91120385.9
(22) Anmeldetag: 28.11.1991
(51) Int. Cl.: C07C 7/08

(54) **Verfahren zur Abtrennung von Aromaten aus Kohlenwasserstoffgemischen beliebigen Aromatengehaltes**
Process for separating aromatics from hydrocarbon mixtures of any given aromatic content
Procédé pour la séparation de composés aromatiques de mélanges d'hydrocarbures de n'importe quelle teneur en composés aromatiques

(30) Priorität: 23.01.1991 DE 4101848
(43) Veröffentlichungstag der Anmeldung: 29.07.1992
(73) Patentinhaber: Krupp Koppers GmbH, D-45143 Essen (DE)
(72) Erfinder: Skatulla, Luzian, W-4330 Mülheim/Ruhr (DE); Schneider, Hans-Christoph, W-4320 Hattingen (DE); Vollmer, Hans-Jürgen, Dr., W-4300 Essen (DE)

(56) Entgegenhaltungen:
- EP-A- 0 085 572
- EP-A- 0 155 992
- EP-A- 0 379 021
- US-A- 4 447 318

## Beschreibung

Die Erfindung betrifft ein Verfahren zur Abtrennung von Aromaten aus Kohlenwasserstoffgemischen beliebigen Aromatengehaltes, die als nichtaromatische Bestandteile insbesondere Paraffine, Cycloparaffine, Olefine, Diolefine sowie organische Schwefelverbindungen enthalten können, durch Extraktivdestillation, bei der N-substituierte Morpholine,deren Substituenten nicht mehr als sieben C-Atome aufweisen, als selektives Lösungsmittel verwendet werden, wobei die nichtaromatischen Bestandteile des als Einsatzprodukt dienenden Kohlenwasserstoffgemisches als Raffinat über Kopf aus der Extraktivdestillationskolonne abdestilliert werden, während die Aromaten zusammen mit dem verwendeten Lösungsmittel als Extrakt aus dem Sumpf der Extraktivdestillationskolonne abgezogen werden und wobei das Raff inat zwecks Rückgewinnung der in ihm vorhandenen Lösungsmittelreste in einer separaten Raffinatdestillationskolonne destilliert wird.

Das vorstehend beschriebene Aromatengewinnungsverfaren ist bereits seit einer ganzen Reihe von Jahren bekannt und hat sich, insbesondere unter Verwendung von N-Formylmorpholin als selektivem Lösungsmittel, in der Zwischenzeit in der Praxis auf verschiedenen großtechnischen Anlagen gut bewährt. Hierbei wird normalerweise das aus der Extraktivdestillationskolonne abgezogene Sumpfprodukt in eine nachgeschaltete Abtreiberkolonne eingeleitet, in der die in ihm als Extrakt enthaltenen Aromaten destillativ vom Lösungsmittel abgetrennt werden. Das Lösungsmittel wird sodann aus dem Sumpf der Abtreiberkolonne abgezogen und zur Wiederverwendung in die Extraktivdestillationskolonne zurückgeführt. Hierbei erfolgt aus verfahrenstechnischen Gründen die Einleitung bzw. Wiedereinleitung des Lösungsmittels normalerweise am Kopf der Extraktivdestillationskolonne. Dadurch läßt es sich jedoch praktisch nicht vermeiden, daß das anfallende Raffinat noch gewisse Lösungsmittelreste enthält, wobei der Lösungsmittelgehalt im Raffinat bis zu 2 Gew.-% betragen kann. Aus Wirtschaftlichkeitsgründen und im Hinblick auf die Gewinnung eines möglichst reinen Raffinates ist es jedoch unerläßlich, diesen Lösungsmittelanteil im Raffinat möglichst weitgehend zurückzugewinnen.

Es ist deshalb üblich, das als Kopfprodukt aus der Extraktivdestillationskolonne abgezogene Raffinat in eine separate Destillationskolonne einzuleiten, in der die Kohlenwasserstoffe des Raffinates vom Lösungsmittel destillativ abgetrennt werden. Da die hierbei als Kopfprodukt gewonnenen Kohlenwasserstoffe des Raffinates in der Praxis meist nur noch einen Lösungsmittelgehalt von <1 ppm aufweisen dürfen, erfordert diese destillative Trennung allerdings einen relativ hohen apparativen Aufwand und Energieverbrauch.

Um den Energieverbrauch herabzusetzen, ist deshalb in der DE-OS 34 09 030 bereits ein Verfahren der gattungsgemäßen Art vorgeschlagen worden, bei dem die Destillation des als Kopfprodukt der Extraktivdestillation anfallenden Raffinates unter solchen Bedingungen betrieben wird, daß das dabei anfallende Sumpfprodukt noch einen Lösungsmittelgehalt von 20 bis 75 Gew.-% aufweist. Anschließend wird dieses Sumpfprodukt in einem Scheidebehälter in eine schwere und eine leichte Phase getrennt, wobei die lösungsmittelreiche schwere Phase in die Extraktivdestillationskolonne und die lösungsmittelarme leichte Phase in die Raffinatdestillationskolonne zurückgeführt wird. Mit dieser Methode gelingt es zwar, den Energiebedarf für die Nachreinigung des Raffinates herabzusetzen. Es ist jedoch in diesem Falle ein zuätzlicher Scheidebehälter für die Trennung von schwerer und leichter Phase erforderlich, was natürlich einen zusätzlichen apparativen Aufwand bedeutet.

Im Patent:US-A-4 447 318 wird vorgeschlagen, für die Trennung der Diolefine von Olefinen und der Olefine von Paraffinen,-nicht aber der Aromaten von Nichtaromaten -,den Energieinhalt des Lösungsmittels für eine vollständige Verdampfung des Einsatzproduktes zu nutzen und das Einsatzprodukt dampfförmig in die Extraktivdestillationskolonne einzuleiten.Für die vollständige Verdampfung werden zwei Wärmeaustauscher mit jeweils unterschiedlichem Druckniveau und ein Verdichter für die Verdichtung des im 2. Wärmeaustauscher bei niedrigerem Druck verdampften Einsatzproduktes benötigt.Die Dampfströme aus den beiden Wärmeaustauschern werden zusammengeführt und gemeinsam der Extraktivdestillation zugeführt.

Die Erfindung verfolgt das Ziel einer weiteren Ausgestaltung des Verfahrens der eingangs genannten Art, durch die die Reinheit der aus dem Extrakt abgetrennten Aromaten verbessert werden soll. Außerdem soll die Aufarbeitung des gewonnenen Raffinates nach Möglichkeit vereinfacht werden, ohne daß die Reinheit der daraus gewonnenen Nichtaromaten beeinträchtigt wird.

Das der Lösung dieser Aufgabe dienende Verfahren ist erfindungsgemäß dadurch gekennzeichnet, daß das Einsatzkohlenwasserstoffgemisch vor dem Eintritt in die Extraktivdestillationskolonne im indirekten Wärmeaustausch mit dem von der Abtreiberkolonne kommenden heißen Lösungsmittel bis auf eine Temperatur zwischen 130 und 150°C erhitzt und in einem Trennbehälter durch Entspannung in eine flüssige und eine dampfförmige Phase zerlegt wird, die getrennt voneinander in die Extraktivdestillationskolonne eingeleitet werden,wobei die Einleitungsstelle für die dampfförmige Phase unterhalb der Einleitungsstelle für die flüssige Phase liegt.

Die Lage der Aufgabestellen wird in Abhängigkeit von der Zusammensetzung des Einsatzkohlenwasserstoffgemisches ermittelt.

Das heißt,im Gegensatz zum US-A-447 318 wird also erfindungsgemäß das Einsatzprodukt erhitzt,nach einer Entspannung nur teilweise verdampft und die flüssige und dampfförmige Phase getrennt in die Extraktivdestillationskolonne eingeführt, wobei die Dampfphase unterhalb der Aufgabestelle für die Flüssigphase eingeleitet und dadurch eine Verbesserung der Benzolreinheit erreicht wird, ohne daß der Energiebedarf sich dabei erhöht.Die Verbesserung der Benzolreinheit durch die Dampfeinleitung unterhalb der Aufgabe für die Flüssigphase ist der Grundgedanke der Erfindung.Gleichzeitig wird eine apparative Vereinfachung sichergestellt.

Gemäß einer bevorzugten Ausführungsform des erfindungs gemäßen Verfahrens wird dabei gleichzeitig das bei der Raffinatdestillation anfallende Sumpfprodukt mit einem Lösungsmittelgehalt von 1,5 bis 2,5 Gew.-% in die Extraktivdestillationskolonne wiedereingeleitet, wobei das Sumpfprodukt als Rückfluß auf den Kopf der Extraktivdestillationskolonne aufgegeben wird.

Das heißt, im Gegensatz zu der in der DE-OS 3409030 beschriebenen Arbeitsweise wird hier auf eine Auftrennung des Sumpfproduktes in einem Phasenscheider in eine schwere und eine leichte Phase verzichtet, so daß sich der apparative Aufwand entsprechend verringert. Die erfindungsgemäße Arbeitsweise ist dabei deshalb nicht naheliegend, weil man bisher immer davon ausgegangen ist, daß ein Rückfluß in der Extraktivdestillationskolonne möglichst vermieden bzw. klein gehalten werden sollte, um eine unnötige Verdünnung des Lösungsmittels durch zurückgeführte Kohlenwasserstoffe des Raffinates sowie die Ausbildung von zwei Flüssigphasen unterschiedlicher Dichte auf den oberen Böden der Extraktivdestillationskolonne zu vermeiden.

Die praktischen Erfahrungen bei der Anwendung des erfindungsgemäßen Verfahrens haben jedoch gezeigt, daß diese Befürchtungen nicht gerechtfertigt waren.

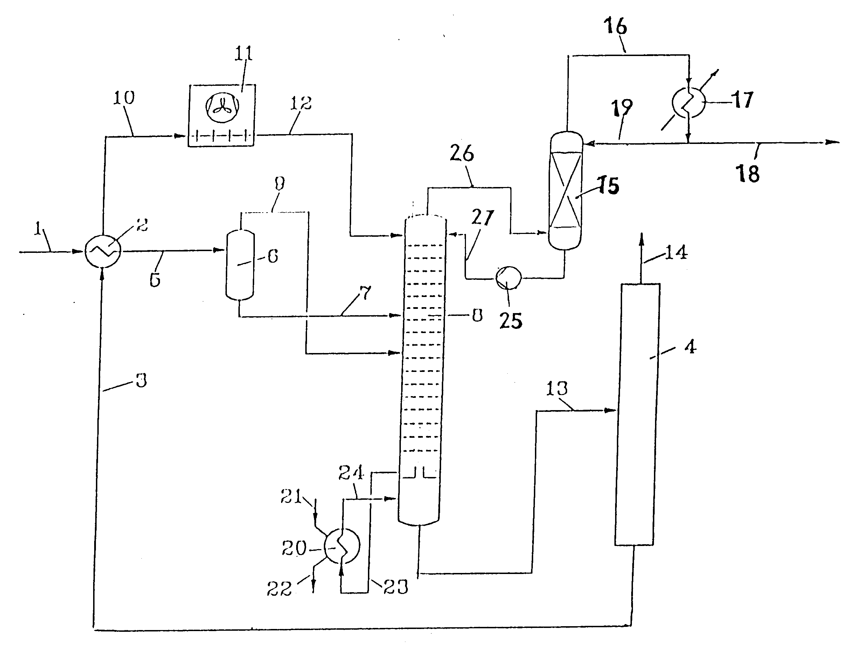

Weitere Einzelheiten des erfindungsgemäßen Verfahrens sollen an Hand des in der Abbildung dargestellten Fließschemas eräutert werden. Das Fließschema enthält dabei nur die für die Verfahrenserläuterung unbedingt notwendigen Anlagenteile, während Nebeneinrichtungen, wie beispielsweise Pumpen, Umlaufkocher, Wärmetauscher, Meß- und Regeleinrichtungen etc., nicht dargestellt sind.

Bei dem in der Abbildung dargestellten Fließschema wird das Einsatzkohlenwasserstoffgemisch über die Leitung 1 in den Wärmetauscher 2 eingeleitet und dort im indirekten Wärmeaustausch mit dem von der Abtreiberkolonne 4 kommenden heißen Lösungsmittel, das über die Leitung 3 zugeführt wird, bis auf eine Temperatur zwischen 130 und 150°C erhitzt. Bei dem im Fließschema dargestellten Ausführungsbeispiel wird das erhitzte Einsatzkohlenwasserstoffgemisch anschließend über die Leitung 5 in den Trennbehälter 6 eingeleitet, in dem es durch Entspannung in eine flüssige und eine dampfförmige Phase zerlegt wird. Die flüssige Phase wird hierbei über die Leitung 7 in den mittleren Teil der mit Böden versehenen Extraktivdestillationskolonne 8 eingeleitet. Beispielsweise kann die Einleitung bei einer Gesamtbodenzahl der Kolonne von 55 Böden in Höhe des 24. Bodens von oben erfolgen. Gleichzeitig wird die dampfförmige Phase über die Leitung 9 unterhalb der Aufgabestelle für die flüssige Phase in die Extraktivdestillationskolonne 8 eingeleitet. Im vorliegenden Falle kann die Einleitung der dampfförmigen Phase beispielsweise 6 Böden unterhalb der Aufgabestelle für die flüssige Phase erfolgen. Wie bereits weiter oben erwähnt wurde, kann das erfindungsgemäße Verfahren gegebenenfalls auch in der Weise durchgeführt werden, daß das vom Wärmetauscher 2 kommende heiße Einsatzkohlenwasserstoffgemisch über die Leitung 5 unmittelbar in den mittleren Teil der Extraktivdestillationskolonne 8 eingeleitet wird.

In dieser Kolonne erfolgt die Auftrennung des Einsatzkohlenwasserstoffgemisches unter dem Einfluß des Lösungsmittels in an sich bekannter Weise. Das vom Wärmetauscher 2 kommende Lösungsmittel gelangt hierbei über die Leitung 10 in den Luftkühler 11, in dem es gegebenenfalls die notwendige Abkühlung erführt, um anschließend mit einer Temperatur zwischen 100 und 110°C über die Leitung 12 in die Extraktivdestillationskolonne 8 eingeleitet zu werden. Das eingeleitete Lösungsmittel fließt über die Böden dieser Kolonne nach unten herab, wobei es die dampfförmigen Aromaten aufnimmt. Das flüssige Sumpfprodukt, das aus dem Lösungsmittel und den darin gelösten Aromaten besteht, wird über die Leitung 13 aus der Extraktivdestillationskolonne 8 abgezogen und in die Abtreiberkolonne 4 eingeleitet, in der dieses vielfach auch als Extrakt bezeichnete Sumpfprodukt in seine Bestandteile zerlegt wird. Auf konstruktive Einzelheiten der Abtreiberkolonne 4 braucht hier nicht näher eingegangen zu werden, da die Aufarbeitung des Extraktes der Extraktivdestillation nicht Gegenstand der vorliegenden Erfindung ist. Die Aromaten werden als Kopfprodukt aus der Abtreiberkolonne 4 über die Leitung 14 abgezogen, während sich das aromatenfreie Lösungsmittel im Sumpf dieser Kolonne sammelt und über die Leitung 3 dem Wärmetauscher 2 zugeführt werden kann.

Die nichtaromatischen Kohlenwasserstoffe des Einsatzkohlenwasserstoffgemisches, die die Raffinatphase bilden, steigen währenddessen in der Extraktivdestillationskolonne 8 dampfförmig nach oben. Damit aus diesen nichtaromatischen Kohlenwasserstoffen die Lösungsmittelreste entfernt werden können, wird die Raffinatphase über Kopf aus der Extraktivdestillationskolonne 8 abgezogen und über die Leitung 26 in die sogenannte Raffinatdestillationskolonne 15 eingeleitet, die ebenfalls mit Böden oder sonstigen Einbauten versehen sein kann. Die von den Lösungsmittelresten befreiten nichtaromatischen Kohlenwasserstoffe ent -weichen dampfförmig über Kopf aus der Raffinatdestillationskolonne 15 und gelangen über die Leitung 16 in den Kühler 17, in dem die Kohlenwasserstoffe kondensiert werden. Die Hauptmenge der flüssigen Nichtaromaten wird anschliessend über die Leitung 18 aus dem Verfahren abgezogen und seiner weiteren Verwendung zugeführt, während ein kleiner Teilstrom über die Leitung 19 als Rückfluß am Kopf in die Raffinatdestillationskolonne 15 wiedereingeleitet wird. Die Rückflußmenge wird dabei so eingestellt, daß die gewonnenen Nichtaromaten den gewünschten Reinheitsgrad aufweisen. Vorzugsweise wird hierbei mit einem Rückflußverhältnis gearbeitet, das bei 0,5 liegt. Das anfallende Sumpfprodukt wird über die Leitung 27 aus der Raffinatdestillationskolonne 15 abgezogen und mittels der Pumpe 25 zur Extraktivdestillationskolonne 8 zurückgefördert, in die es am Kopf als Rückfluß wiedereingeleitet wird.

Wie bereits weiter oben erwähnt wurde, werden bei einer bevorzugten Ausführungsform des erfindungsgemäßen Verfahrens die Destillationsbedingungen in der Raffinatdestillationskolonne 15 so eingestellt, daß das Sumpfprodukt dieser Kolonne mit einem Lösungsmittelgehalt von 1,5 bis 2,5 Gew.-% über die Leitung 27 zur Extraktivdestillationskolonne 8 zurückgefördert wird.

Für die Beheizung ist am Sumpf der Extraktivdestillationskolonne 8 der Umlaufkocher 20 vorgesehen, in dem das umlaufende Sumpfprodukt im indirekten Wärmeaustausch mit Dampf erhitzt wird. Die Leitungen 21 und 22 dienen hierbei der Zu- und Abführung des Dampfes und die Leitungen 23 und 24 der Zu- und Abführung des Sumpfproduktes. Selbstverständlich können an der Extraktivdestillationskolonne 8 auch noch weitere Seitenkocher zur zusätzlichen Kolonnenbeheizung angeordnet sein. Da die Kolonnenbeheizung jedoch nicht Gegenstand der vorliegenden Erfindung ist, braucht auf diese Ausführungsformen nicht näher eingegangen zu werden.

Die Wirksamkeit des erfindungsgemäßen Verfahrens wird durch die Ergebnisse der nachfolgenden Versuchsreihe belegt. Als Einsatzkohlenwasserstoffgemisch wurde hierbei ein Pyrolysebenzin verwendet, wobei die Durchsatzkapazität der Anlage ca. 14600 kg/h betrug. In Teil a) der Versuchsreihe (Versuch 1) wurde in der bisher üblichen Weise ohne Erhitzung des Einsatz-Kohlenwasserstoffgemisches gearbeitet. Teil b) betrifft demgegenüber die Anwendung des erfindungsgemäßen Verfahrens, wobei bei Versuch 2 das erhitzte Einsatzkohlenwasserstoffgemisch direkt in die Exraktivdestillationskolonne eingeleitet wurde. Bei Versuch 3 wurde dagegen das erhitzte Einsatzkohlenwasserstoffgemisch zunächst in eine flüssige und eine dampfförmige Phase zerlegt und beide Phasen getrennt voneinander in die Extraktivdestillationskolonne eingeleitet. Die erzielten Versuchsergebnisse sind in der nachfolgenden Tabelle zusammengefaßt.

**Tabelle**

| Versuch Nr. | Zulauf temperatur Einsatzprodukt °C | Zulaufboden (v.oben) Dampf/Flüssigkeit | Nichtaromaten im Benzol ppm | Lösungsmittel in Nichtaromaten (Raffinat) ppm | Fremd-energ. bedarf Gcal/h |
|---|---|---|---|---|---|
| | | | | | |

| a) Extraktivdestillation nach dem Stand der Technik gemäß DE-OS 34 09 030 | | | | | |
|---|---|---|---|---|---|
| 1 | 80 | - / 24 | 170 | <3.10⁻³ | 1.806 |

| b) Extraktivdestillation nach der Erfindung | | | | | |
|---|---|---|---|---|---|
| 2 | 130 | - / 24 | 123 | <3.10⁻³ | 1.803 |
| 3 | 130 | 30 / 20 | 98 | <3.10⁻³ | 1.803 |

Die vorliegenden Versuchsergebnisse zeigen eindeutig, daß bei Anwendung des erfindungsgemäßen Verfahrens eine deutlich verbesserte Reinheit des gewonnenen Benzols erreicht werden konnte, ohne daß der Fremdenergiebedarf höher lag als bisher und ohne daß es zu einer Verschlechterung der Reinheit der gewonnenen Nichtaromaten kommt. Da außerdem in diesem Falle auf eine Phasentrennung des Sumpfproduktes der Raffinatdestillationskolonne in einem separaten Phasenscheider verzichtet wurde, tritt zusätzlich eine deutliche Ersparnis an Anlage- und Betriebskosten ein.

## Patentansprüche

1. Verfahren zur Abtrennung von Aromaten aus Kohlenwasserstoffgemischen beliebigen Aromatengehaltes, die als nichtaromatische Bestandteile insbesondere Paraffine, Cycloparaffine, Olefine, Diolefine sowie organische Schwefelverbindungen enthalten können, durch Extraktivdestillation, bei der N-substituierte Morpholine, deren Substituenten nicht mehr als sieben C-Atome aufweisen, als selektives Lösungsmittel verwendet werden, wobei die nichtaromatischen Bestandteile des als Einsatzprodukt dienenden Kohlenwasserstoffgemisches als Raffinat über Kopf aus der Extraktivdestillationskolonne (8) abdestilliert werden, während die Aromaten zusammen mit dem verwendeten Lösungsmittel als Extrakt (13) aus dem Sumpf der Extraktivdestillationskolonne abgezogen werden und der Extrakt in einer Abtreiberkolonne (4) in seine Bestandteile zerlegt wird und wobei das Raffinat (26) zwecks Rückgewinnung der in ihm vorhandenen Lösungsmittelreste in einer separaten Raffinatdestillationskolonne (15) destilliert wird, dadurch gekennzeichnet, daß das Einsatzkohlenwasserstoffgemisch (1) vor dem Eintritt in die Extraktivdestillationskolonne im indirekten Wärmeaustausch (2) mit dem von der Abtreiberkolonne kommenden heißen Lösungsmittel bis auf eine Temperatur zwischen 130 und 150°C erhitzt und in einem Trennbehälter (6) durch Entspannung in eine flüssige und eine dampfförmige Phase zerlegt wird,die getrennt voneinander in diese Kolonne eingeleitet werden,wobei die Einleitungsstelle für die dampfförmige Phase unterhalb der Einleitungsstelle für die flüssige Phase liegt.

2. Verfahren nach dem Anspruch 1,dadurch gekennzeichnet, daß das bei der Raffinatdestillation anfallende Sumpfprodukt mit einem Lösungsmittelgehalt von 1,5 bis 2,5 Gew-% in die Extraktivdestillationskolonne wiedereingeleitet wird, wobei das Sumpfprodukt als Rückfluß auf den Kopf der Extraktivdestillationskolonne aufgegeben wird.

## Claims

1. Method of separating aromatics from hydrocarbon mixtures of arbitrary aromatics content, which can contain especially paraffins, cycloparaffins, olefins, diolefins and organic sulphur compounds as non-aromatic constituents, by extractive distillation in which N-substituted morpholines, whose substituents have no more than seven C atoms, are used as a selective solvent, the non-aromatic constituents of the hydrocarbon mixture used as feedstock being distilled off as raffinate at the top of the extractive distillation column (8), while the aromatics, together with the solvent used, are withdrawn as extract (13) from the bottom of the extractive distillation column and the extract is split into its constituents in a stripping column (4), and the raffinate (26) being distilled in a separate raffinate distillation column (15) for the purpose of recovering the solvent residues present therein, characterised in that, before it enters the extractive distillation column, the mixed hydrocarbon feedstock (1) is heated to a temperature of between 130 and 150°C by indirect heat exchange (2) with the hot solvent coming from the stripping column and in a separation vessel (6) is split, by expansion, into a liquid phase and a vapour phase, which are introduced separately into this column, the point at which the vapour phase is introduced being below the point at which the liquid phase is introduced.

2. Method according to Claim 1, characterised in that the bottom product obtained in the raffinate distillation is reintroduced into the extractive distillation column with a solvent content of 1.5 to 2.5% by weight, the bottom product being fed into the top of the extractive distillation column as reflux.

## Revendications

1. Procédé pour la séparation de composés aromatiques de mélanges d'hydrocarbures de n'importe quelle teneur en composés aromatiques, qui peuvent contenir tant que constituants non aromatiques en particulier des paraffines, des cycloparaffines, des oléfines, des dioléfines ainsi que des composés soufrés organiques, par distillation extractive, lors de laquelle l'on utilise en tant que solvant sélectif des morpholines substituées en N, dont les substituants ne présentent pas plus de sept atomes de C, les constituants non aromatiques du mélange d'hydrocarbures servant en tant que produit d'emploi étant éliminés en tant que produit raffiné par distillation par la tête hors de la colonne de distillation extractive (8), alors que les composés aromatiques sont retirés conjointement au solvant utilisé en tant qu'extrait (13) hors du bas de colonne de la colonne de distillation extractive et l'extrait étant décomposé en ses constituants dans une colonne de strippage (4), et le produit raffiné (26) étant distillé en vue de la récupération des restes de solvant qui s'y trouvent dans une colonne séparée de distillation du produit raffiné (15), caractérisé en ce que le mélange d'hydrocarbures d'emploi (1) est chauffé avant l'entrée dans la colonne de distillation extractive dans un échange thermique indirect (2) avec le solvant chaud provenant de la colonne de strippage jusqu'à une température comprise entre 130 et 150°C, et en ce qu'il est décomposé par détente dans un récipient de séparation (6) en une phase liquide et une phase vapeur, qui sont introduites séparées l'un de l'autre dans cette colonne, le point d'admission pour la phase vapeur se situant en-dessous du point d'admission pour la phase liquide.

2. Procédé selon la revendication 1, caractérisé en ce que le produit de bas de colonne qui se forme lors de la distillation du produit raffiné avec une teneur en solvant de 1,5 à 2,5 % en poids est de nouveau introduit dans la colonne de distillation extractive, le produit de bas de colonne étant admis en tant que reflux à la tête de la colonne de distillation extractive.
